Europäisches Patentamt

European Patent Office

Office européen des brevets

(11) Publication number: **0 428 245 A1**

# EUROPEAN PATENT APPLICATION

(21) Application number: 90307169.4

(22) Date of filing: 29.06.90

(51) Int. Cl.5: **A61L 2/26, C12Q 1/22**

(30) Priority: 16.11.89 US 437543

(43) Date of publication of application:
22.05.91 Bulletin 91/21

(84) Designated Contracting States:
**DE FR GB IT**

(71) Applicant: **PYMAH CORPORATION**
**89 Route 206**
**Somerville New Jersey 08876(US)**

(72) Inventor: **Hanafin, Paul**

**deceased(US)**

(74) Representative: **Coxon, Philip et al**
**Eric Potter & Clarkson St. Mary's Court St.**
**Mary's Gate**
**Nottingham NG1 1LE(GB)**

(54) Integrator test pack for steam sterilization.

(57) A disposable sterilization test pack for determining the efficacy of sterilizing equipment comprises a porous base pad having a cavity for receiving a sterilization integrator. Porous pads are disposed on opposite sides of the base pad to retain the integrator within the cavity. An impermeable layer of laminate is disposed against one of the porous pads to confine the passage of gas through the pack to the edges of the pack and to the porous pad which is not covered by the impermeable layer.

Fig. 2

This invention relates generally to packs for testing the efficacy of a sterilization system. More particularly, this invention relates to disposable packc for testing the effectiveness of sterilization processes. More specifically, the invention relates to a disposable test pack which can be used with a sterilizatior integrator to determine the effectiveness of a sterilization system cycle. The present invention is particularly, though not exclusively, useful for testing steam autoclaves that are used for sterilizing hospital and medical equipment.

As is well known, the sterilization of hospital equipment may be accomplished by exposing the equipment to steam, using a device known as an autoclave. To accomplish this, the equipment to be sterilized is first placed within the autoclave. A vacuum is then drawn and steam is introduced into the autoclave to permeate the equipment and sterilize it.

As can well be appreciated, sterilization equipment must be periodically tested to ensure that the sterilizaton process is, in fact, efficacious. Under one accepted protocol, such a test is conducted by subjecting spores of living micro-organisms to the sterilization process and subsequently observing whether the spores remain viable. The commonly used test spore of choice for this procedure is Bacillus Stearothermophilus. Another method for testing the efficacy of a sterilization process is to subject a chemical indicator to the sterilization process to indicate whether effective conditions for sterilization have been met. Yet a third method of testing a sterilization device, such as an autoclave, is to subject a chemical integrator to the sterilization process. With an integrator, certain sterilization factors (e.g., time and temperature) are measured simultaneously to indicate whether an effective combination of these factors is attained for adequate sterilization to have been achieved. To ensure that the sterilization test provides a sufficient challenge for the sterilization equipment, the test micro-organisms, chemical indicator, or integrator must be protected as well or better than micro-organisms would ordinarily be protected if lodged in the most inaccessible recesses of the hospital equipment to be sterilized.

Several procedures have been proposed to test the efficacy of steam sterilization equipment. Typical of these, and perhaps the best known and most widely used, is the procedure published by the Association for the Advancement of Medical Instrumentation (A.A.M.I.). According to this procedure, freshly-laundered, all-cotton towels are folded by hospital personnel and stacked to construct a test pack into which a biological indicator is imbedded. This pack is then subjected to a sterilization cycle and the indicator is subsequently observed to determine whether the cycle has been efficacious.

Although the A.A.M.I. test procedure is adequate for its intended purpose, the procedure is labor intensive and results in a relatively bulky, cumbersome test pack. Moreover, the A.A.M.I. method requires relatively lengthy post-test periods (e.g. days) before test results can be obtained. This is so because it uses biological indicators rather than integrators. Not surprisingly, several devices have been proposed to mimic the A.A.M.I. protocol. For example, U.S.-A-4,636,472, recognized the need to provide a biological test pack that was small, compact, easily handled by hospital personnel, and which sufficiently challenged the sterilization equipment. However, while providing for the use of biological or chemical indicators, U.s.-A-4,fi36,472 to Bruso did not contemplate or provide for the use of an integrator to indicate sterilization cycle efficacy due to the wide acceptance of available biological indicators.

Use of an integrator in a sterilization test pack, however, has significant advantages over use of the more conventional, A.A.M.I.-recognized biological indicator. Specifically, results of the sterilization equipment test may be obtained from an integrator almost immediately after completion of the test sterilization cycle. In contrast, sterilization test results from a biological indicator typically take up to several days to obtain. One disadvantage of delays in obtaining test results is that hospitals do not normally possess a large enough inventory of equipment to permit shelving sterilized equipment for the length of time it takes to receive the sterilization test results. Thus, equipment which has undergone a faulty sterilization cycle could potentially be distributed for use before test results indicating cycle inadequacy could be obtained. Additionally, results from biological indicators are susceptible to greater statistical variations than are the relatively standardized, consistent results which are obtained from integrators.

Several studies have been conducted which reveal the extent of the statistical variations inherent in biological indicators. For example, a Chemical and Biological Steam Sterilization Indicator Evaluation conducted by Sterilization Technical Services Inc. (Litsky, 1982), indicated that biological indicators may yield over eighty percent false negatives under certain lab conditions. In contrast, the integrators evaluated by Litsky yielded no false negatives. In addition to the relative advantages integrators have over biological indicators, as discussed above, integrators also possess certain advantages over chemical indicators. For example, crucial to steam cycle sterilization efficacy is the interdependence of cycle time, temperature and humidity. While many existing chemical indicators do provide test results relatively quickly, in the manner of integrators, they cannot be calibrated to provide a consistent indication of the efficacy of the steam sterilization equipment for all cycle time-temperature-humidity combinations. Stated differently, standard chemical indicators may provide an accurate indication of sterilization cycle efficacy for some combinations of temperature, time, and humidity, but not for others. In contrast, integrators provide a quick, consistent and standardized indication of sterilization equipment efficacy for substantially all sterilization cycle time-temperature-humidity combinations.

It is an object of this invention to provide an improved disposable test pack for testing the effectiveness of sterilization process.

According to one aspect of this invention there is provided a disposable pack for testing the effectiveness of a sterilization process, which comprises:
a plurality of gas-permeable porous sheets, each of said sheets having a hole therethrough and juxtaposed to form a base pad having a top, a bottom, an edge therebetween, and a cavity established by the alignment of said holes for receiving an integrator therein;
a porous top pad having an edge and disposed against said top of said base pad with the edge of said top pad being substantially flush with said edge of said base pad; and
a porous bottom pad having an edge and disposed against said bottom of said base pad with said edge of said bottom pad being substantially flush with said edge of said base pad.

According to another aspect of this invention there is provided a disposable pack for testing the effectiveness of a sterilization process, which comprises:
an integrator;
a plurality of gas-permeable porous sheets, each of said sheets having a hole therethrough and juxtaposed to form a base pad having a top, a bottom, an edge therebetween, and a cavity established by the alignment of said holes for receiving said integrator therein;
a porous top pad having an edge and disposed against said bottom of said base pad with said edge of said bottom pad being substantially flush with said edge of said base pad.
a porous bottom pad having an edge and disposed against said bottom of said base pad with said edge of said bottom pad being substantially flush with said edge of said base pad.

The top pad may comprise a single gas-permeable porous sheet, and may further comprise a gas-impermeable layer disposed against said sheet to substantially direct the passage of gas through said edges of said top pad, said base pad, and said bottom pad, and through said bottom pad.

According to another aspect of this invention there is provided a disposable integrator test pack for testing the efficacy of a sterilization process, which comprises:
a plurality of gas-permeable porous sheets, each of said sheets having a hole therethrough and juxtaposed to form a base pad having a top surface, a bottom surface, an edge therebetween and a cavity established by the alignment of said holes for receiving said integrator therein;
a porous bottom pad having an edge and being disposed against said bottom surface with its edge substantially flush with said edge of said base pad; and
a porous top pad having an edge and a surface disposed against said top surface with its edge substantially flush with said edge of said base pad.

According to another aspect of this invention there is provided a method for testing the efficacy of a steam sterilization cycle comprising the steps of:
stacking a plurality of gas porous sheets having holes therethrough to establish a test pack having a top side, a bottom side, and plurality of edges;
aligning said holes to form a cavity;
placing an integrator within said cavity;
stacking a single gas-impermeable laminate sheet on said top side of said test pack to direct gas flow through said edges and said bottom side;
stacking a single continuous porous sheet on said bottom side to hold said integrator within said cavity;
subjecting said test pack to said steam sterilization process;
removing said integrator from said test pack; and
examining said integrator to assess the efficacy of said sterilization process.

A preferred embodiment of the novel disposable sterilization test pack includes a base pad which comprises a plurality of gas-permeable porous sheets. Each of the porous sheets which comprise the base pad has a hole cut through it. The sheets are stacked to align the holes and form a cavity for receiving a sterilizaton integrator. A top pad and a bottom pad of gas-permeable porous material may be placed on the top and bottom surfaces of the base pad, respectively, to hold the integrator inside the cavity and to impede the flow of gas to the cavity. In a variation of the preferred embodiment, a gas-permeable plastic laminated sheet is placed against the top pad on its surface opposite the base pad to further inhibit gas passage through the cavity, and to direct the flow of gas through the cavity.

When the assembled test pack is subjected to a sterilization cycle, the sterilization integrator, positioned within the cavity of the base pad, may react according to cycle time, temperature, and steam saturation. Consequently, the reaction of the integrator to these factors provides a corresponding indication of cycle efficacy. The test pack can be enveloped with an overwrap material and held together with a tape. The tape may have an indicating ink imprinted on it to show whether the pack has been subjected to a sterilization cycle.

Thus, by incorporating integrators as the sterilization sensors, the preferred embodiment of the present invention satisfies the need for a pre-assembled composite sterilization test pack which is convenient to handle, which will sufficiently challenge the sterilization equipment during a sterilization cycle, and which will provide for quick, standardized sterilization test results for substantially all cycle time, temperature, and humidity combinations.

Accordingly, it is an advantage of the present invention that it provides a pre-assembled sterilization test pack which tests the efficacy of steam sterilization equipment by challenging the accessibility of steam to the sterilization condition sensing device, and by providing a requisite level of thermal insulation for the device. Yet another advantage of the present invention is that it provides a test pack that is small, compact, and easily handled by hospital personnel. Still another advantage of the present invention is that it provides a test pack that yields test results relative quickly, and is accurate over a wide range of sterilization conditions. Another advantage of the present invention is that it provides a test pack which is convenient to use, easy to interpret, cost effective and easily manufactured.

Reference is now made to the accompanying drawings in which:

Figure 1 is a side cross-sectional view of a steam sterilizer showing the test pack of the present invention located therein;

Figure 2 is an exploded perspective view of the present invention;

Figure 3 is a perspective view of the sterilization test pack folded and ready for use in sterilization equipment;

Figure 4 is a side cut-away cross-sectional view of the integrator as seen along the line 4-4 of Figure 2;

Figure 5 is an exploded perspective view of the contents of an alternate embodiment of the sterilization test pack;

Figure 6 is an exploded perspective view of the contents of another embodiment of the sterilization test pack; and

Figure 7 is an exploded perspective view of the contents of yet another embodiment of the sterilization test pack.

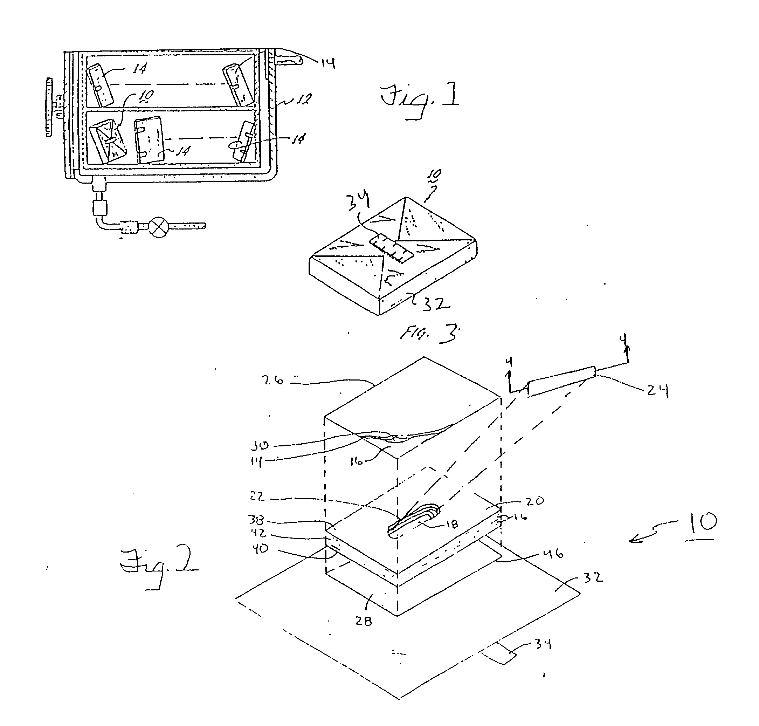

Figure 1 shows the test pack 10 of the present invention placed inside a steam sterilizer 12 for testing the efficacy of sterilizer 12. Also shown placed in sterilizer 12 are various bundles 14 of medical equipment which require sterilization.

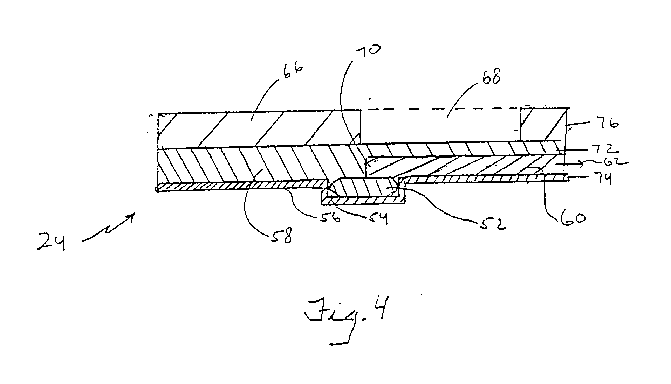

Referring now to Figure 2, it can be seen that test pack 10 comprises a base pad 20, a top pad 26, and a bottom pad 28 generally oriented with respect to each other as shown. With regard to base pad 20 and reference to Figure 2, it will be appreciated that base pad 20 is constructed by stacking together several gas-permeable porous sheets 16. As seen in Figure 2, these sheets 16 can have die-cut configurations to form holes 18, which, when sheets 16 are stacked together, form a cavity 22. It will be appreciated by the skilled artisan that, depending on the configuration of hole 18, cavity 22 can be formed to conform to the size and shape of the particular chemical integrator being used. In the preferred embodiment, sheet 16 comprises a nonwoven, gas-permeable porous material such as heavyweight index paper which is well known in the pertinent art.

As seen in Figure 2, base pad 20 is generally box-shaped and has a top surface 38, a bottom surface 40, and edge 42 defined between them. As will be appreciated by the skilled artisan, although pad 20 is shown in Figure 2 as being box-shaped, the particular shape of base pad 20 can be modified depending on the desires of the manufacturer. As will be more clearly discussed below, however, the top surface 38 and the bottom surface 40 are preferably flat in order to engage with top pad 26 and bottom pad 28.

Still referring to Figure 2, it is seen that the cavity 22 is configured by holes 18 to receive an integrator 24. In the preferred embodiment, an integrator of the type described in U.S. Patent No. 4,636,472 is appropriate for use in the present invention.

In addition to base pad 20, test pack 10 also includes a top pad 26. Preferably, top pad 26 comprises a single porous sheet 16 having an edge 44 and in contrast to base pad 20, top pad 26 is not formed with a hole. In the construction of test pack 10, top pad 26 is positioned against top surface 38 of base pad 20 with edge 44 substantially flush with edge 42 of base pad 20. Also, top pad 26 is positioned against base pad 20 such that integrator 24 may be held within the cavity 22. For the same purpose, bottom pad 28 is disposed against bottom surface 40 of base pad 20 with its edge 46 substantially flush with edge 42 of base pad 20. As seen in Figure 2, bottom pad 28 comprises a single porous sheet 16 to form bottom pad 28. Sheet 16 of bottom pad 28 is continuous and, like top pad 26, does not have a hole cut therethrough. For the purpose of geometric description, the box-shaped structures herein described are essentially parallelpipeds.

A non-porous, gas-impermeable layer 30 may be disposed against top pad 26 in any pertinent manner well-known in the art. Layer 30, when so placed against top pad 26, prevents the passage of gas transverse to that portion of the surface of top pad 26 which is in contact with layer 30. Stated differently, layer 30 is positioned against top pad 26 to direct the passage of gas through test pack 10 transverse to the exposed surface of bottom pad 28. Preferably, layer 30 comprises a plastic film that is laminated to top pad 26. It will be appreciated by the skilled artisan, however, that lamination is but one means available to position layer 30 against top pad 26. As implied above, layer 30 may be eliminated. On the other hand, sheet 16 of top pad 26, which supports layer 30, can be eliminated if layer 30 is constructed with sufficient stiffness to support itself.

In accordance with the above description, for the preferred embodiment of the present invention, bottom pad 28 comprises a single sheet 16, with no die cut; base pad 20 comprises about thirty (30) die-cut sheets 16; and top pad 26 is composed of a single continuous sheet 16. Further, layer 30 is preferably laminated across the entire outer surface of sheet 16 of top pad 26. Accordingly, when assembled, test pack 10 comprises, from top to bottom, gas-impermeable layer 30, gas-permeable porous top pad 26 comprising a single continuous sheet 1, gas-permeable base pad 20 comprising about thirty (30) die-cut sheets 16, which form cavity 22; and gas- permeable bottom pad 28, which comprises a single continuous sheet 16.

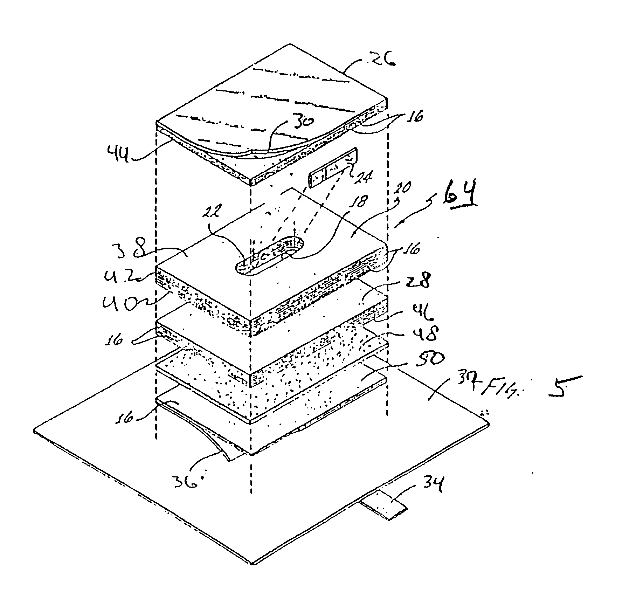

As seen in Figure 3, the integrity of test pack 10 may be maintained by folding a disposable overwrap material 32 around test pack 10 and holding the wrap in place with autoclave tape 34. When test pack 10 is so constructed, it comprises a self-contained unit which can be easily handled by sterilization equipment operators. The present invention envisions the use of a chemical integrator 24, placed within cavity 22, as the cycle efficacy indicating device.

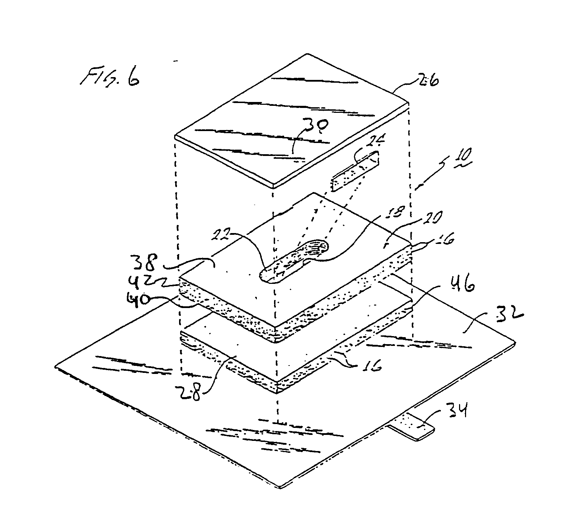

Referring now to Figure 4, it will be seen that integrator 24 comprises a temperature-sensitive chemical 52, which is disposed in cavity 54 of base 56. Base 56 is composed of any material suitable for providing a moisture barrier against steam during the sterilization process. In the preferred embodiment, base 56 is composed of aluminium foil of approximately three (3) mils in thickness. As the skilled artisan will appreciate, the material composition of temperature-sensitive chemical 52 is selected such that chemical 52 melts when predetermined temperature and humidity conditions in cavity 54 have been attained. In the preferred embodiment, temperature-sensitive chemical 52 is composed of an organic material which has a rate of melt that closely follows the death curve of B-stearothermophillis.

As shown in Figure 4, wick 60 is disposed on base 56 with end 70 of wick 60 in contact with chemical 52. Wick 60 is composed of any material suitable for absorbing chemical 52 in its liquid state, as discussed below. A transparentl porous polymeric cover 58 is also disposed on base 56 to cover wick 60 and chemical 52. As shown, the edges 72 of polymeric cover 58 are substantially flush with the edges 74 of base 56. Likewise, a porous paper cover sheet 66 is placed over polymeric cover 58 with edges 76 of cover sheet 66 being substantially flush with the edges 72 and 74 of polymeric cover 58 and base 56, respectively. Cover sheet 66 is formed with a window 68 to permit visual inspection of wick 60. In addition, cover sheet 66 is scaled along window 68 to provide an indication of sterilization cycle efficacy, as discussed more fully below.

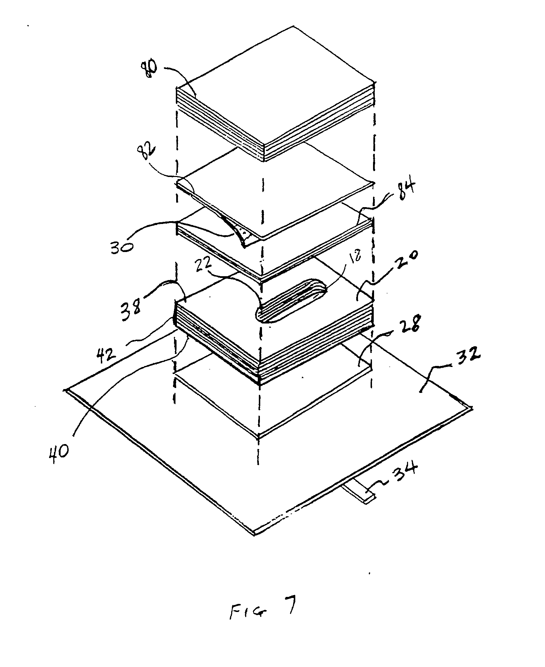

In an alternative embodiment of the present invention, generally designated 64 in Figure 5, the preferred embodiment is altered to further comprise a layer 48 of reticulated foam which is positioned against the exposed surface of bottom pad 28 to position bottom pad 28 between base pad 20 and foam layer 48. Additionally, a cover pad 50 is positioned to place foam layer 48 between bottom pad 28 and cover pad 50. It will be appreciated that layer 48 may or may not be removed and, in either case a gas-impermeable nonporous layer 36 can be laminated against cover pad 50 in a manner similar to the description above for top pad 26 and layer 30.

In yet another embodiment of the present invention, as shown in Figure 6, top pad 26 is constructed solely of gas-impermeable non-porous layer 30. For the integrity of pack 10 and ease of construction, impermeable layer 30 of this alternate embodiment should be of a plastic well known in the art which is sufficiently stiff to provide the support for integrator 24 as it rests in cavity 22. In all important respects, the embodiments shown in Figure 5 and Figure 6 are constructed in the manner described for the preferred embodiment and function essentially in the manner described above for the preferred embodiment.

In still another embodiment of the present invention, the test pack 10 is altered as shown in Figure 7 to comprise a top pad 80 and a porous sheet 82 which are positioned on one side of a gas-impermeable, non-porous middle layer 30. Further, a second gas-permeable porous layer 84 is positioned on the opposite side of middle layer 30. Again, in all important respects, the embodiment shown in Figure 7 is constructed in the manner described for the preferred embodiment and functions essentially in the same manner.

Accordingly, when assembled, this alternative embodiment for test pack 10 comprises, sequentially from top to bottom, gas-permeable top pad 80, a gas-impermeable layer 30, which may or may not include the porous sheet 82, a second gas-ermeable pad 84, gas-permeable base pad 20 and gas-permeable bottom pad 28. Also, it is to be appreciated that gas-impermeable layer 30 and porous layer 84 can be moved from between porous pad 80 and base pad 20 to be situated between base pad 20 and bottom pad 28. With this configuration, test pack 10 will still function in essentially the same manner as previously described.

The skilled artisan will recognize that the present invention is capable of several variations and modifications. For example, top pad 26 may comprise a plurality of sheets 16 as shown in Figure 5, instead of a single sheet 16 as described in the preferred embodiment. The number of sheets 16 used for base pad 20 and bottom pad 28 may be likewise varied. Further, although the preferred dimensions of sheets 16 are approximately 4.5" x 6", these dimensions can be changed to vary the resistance of test pack 10 and meet the particular needs and desires of the operator.

## OPERATION

In the operation of test pack 10 of the present invention, an integrator 24 is placed within the cavity 22 that is formed within porous base pad 20. A porous top pad 26 is positioned against the top surface 38 of base pad 20, and porous bottom pad 28 is positioned against bottom surface 40 of base pad 20 to confine the integrator 24 within the cavity 22. An impermeable layer 30 is positioned against top pad 26 so as to confine the introduction of gas into and evacuation of gas from test pack 10 throughout edges 42, 44 and 46 and transverse to the exposed surface of bottom pad 28. Test pack 10 is then wrapped in disposable wrap 32 and held together by autoclave tape 34 in a manner which allows placement of test pack 10 into a steam sterilizer 12 for testing of sterilizer 12. Test pack 10 is then subjected to the sterilization cycle.

During the sterilization cycle, steam is introduced into a steam sterilizer 12 and enters test pack 10 through edges 42, 44 and 46, and transverse to the exposed surface of bottom pad 28. Moisture inside test pack 10 enters cavity 22. This moisture in turn penetrates integrator 24 through edges 72, 74, and 76 of polymeric cover 58, base 56, and cover sheet 66, respectively, and transverse to cover sheet 66 and porous polymeric cover 58 to enter into cavity 54, where it contacts temperature-sensitive chemical 52 and lowers its melting point. As indicated above, the rate at which chemical 52 melts depends upon both the rate at which moisture penetrates polymeric cover 58 and the temperature inside cavity 54. In response to these two variables, it happens that the rate at which melted chemical 52 migrates across wick 60 in the direction of arrow 62 closely parallels the death rate of the challenge bacteriological test spore of choice (bacillus stearothermophilus) under equivalent temperature and humidity conditions. As indicated above, cover sheet 66 is scaled to provide an indication of sterilization cycle efficacy based on the extent of chemical 52 migration across wick 60 in the direction of arrow 62. Thus, the total amount of chemical 52 which melts is used to indicate whether conditions within sterilizer 12 were sufficient to kill an acceptable percentage of the test spore of choice.

After completion of the test cycle, test pack 10 is removed from sterilizer 12 and integrator 24 is examined to determine the efficacy of the cycle.

While the particular integrator test pack for steam sterilization, as herein shown and disclosed in detaill is fully capable of obtaining the objects and providing the advantages herein before stated, it is to be understood that it is merely illustrative of the presently preferred embodiments of the invention and that no limitations are intended to the details of construction or design herein shown other than as defined in the appended claims.

## Claims

1. A disposable pack for testing the effectiveness of a sterilization process, which comprises:
a plurality of gaspermeable porous sheets, each of said sheets having a hole therethrough and juxtaposed to form a base pad having a top, a bottom, an edge therebetween, and a cavity established by the alignment of said holes for receiving an integrator therein;
a porous top pad having an edge and disposed against said top of said base pad with the edge of said top pad being substantially flush with said edge of said base pad; and
a porous bottom pad having an edge and disposed against said bottom of said base pad with said edge of said bottom pad being substantially flush with said edge of said base pad.

2. A disposable pack according to Claim 1 comprising:
a gas-impermeable layer disposed against said top pad to substantially direct the passage of gas through said edges of said top pad, said base pad, and said bottom pad, and through said bottom pad.

3. A disposable pack according to Claim 1 wherein said top pad comprises:
a plurality of gas-permeable porous sheets, juxtaposed to define a top, a bottom and an edge therebetween for said top pad;

a second porous pad having a top, a bottom and an edge therebetween; and

a gas-impermeable layer disposed between said top pad and said second porous pad to direct the passage of gases through said edge of said second porous pad, said base pad and said bottom pad, and transversely through said bottom pad.

4. A disposable pack according to Claim 2 or 3 wherein said gas-impermeable layer is a plastic laminate.

5. A disposable pack according to any preceding claim comprising a gas-permeable porous overwrap surrounding said pack to maintain the integrity thereof.

6. A disposable pack according to any preceding claim wherein said holes as formed by a die-cut process.

7. A method for testing the efficacy of a steam sterilization cycle comprising the steps of:

stacking a plurality of gas porous sheets having holes therethrough to establish a test pack having a top side, a bottom side, and plurality of edges;

aligning said holes to form a cavity;

placling an integrator within said cavity;

stacking a single gas-impermeable laminate sheet on said top side of said test pack to direct gas flow through said edges and said bottom side;

stacking a single continuous porous sheet on said bottom side to hold said integrator within said cavity;

subjecting said test pack to said steam sterilization process;

removing said integrator from said test pack; and examining said integrator to assess the efficacy of said sterilization process.

8. A method according to Claim 7 comprising the step of forming said holes with a die-cut.

9. A method according to Claim 7 or 8 comprising the step of wrapping said test pack in a gas-permeable overwrap material to maintain the integrity of said test pack.

Fig. 1

FIG. 3

Fig. 2

Fig. 4

FIG. 6

FIG 7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 202 724 (WARNER LAMBERT) — — — | | A 61 L 2/26 |
| A | US-A-4 486 387 (T.A. AUGURT) — — — | | C 12 Q 1/22 |
| A | US-A-4 576 795 (L.H. BRUSO) — — — | | |
| A | EP-A-0 132 107 (VICTORIA UNIVERSITY OF MANCHES-TER) — — — — — | | |

TECHNICAL FIELDS
SEARCHED (Int. Cl.5)

A 61 L

The present search report has been drawn up for all claims

| Place of search | Date of completion of search | Examiner |
|---|---|---|
| The Hague | 22 November 90 | PELTRE CHR. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document